# EUROPEAN PATENT APPLICATION

(11) **EP 4 413 956 A1**
(43) Date of publication of application: **14.08.2024**
(21) Application number: 24155315.5
(22) Date of filing: 01.02.2024
(51) Int. Cl.: A61F 2/30

(54) **JOINT IMPLANT WITH A POROUS STRUCTURE**

(30) Priority: 10.02.2023 US 202363484255 P
(71) Applicant: Waldemar Link GmbH & Co. KG, 22339 Hamburg (DE)
(72) Inventor: LINK, Helmut D., 22339 Hamburg (DE)
(74) Representative: Alatis

(57) **Abstract**

The disclosure includes an implant (100) configured to contact a portion of a bone, the implant including a stem (2) having a proximal section (4), a distal section (6) and a porous portion (8, 8', 8") extending a distance along the stem (2) in a longitudinal direction of the stem (2).

## Description

### FIELD

The present disclosure relates to joint implants for implantation into a bone.

### BACKGROUND

One of the advances in orthopedic surgery is the apposition of bone tissue on implants that provides a strong and durable integration of these implants within a skeletal structure. In general, there are two techniques for anchoring a joint implant in bone tissue. The first technique was developed by Charnley and is based on bone cement (PMMA) as a means for fixation of the implant within a bone cavity. However, the bone cement may be exposed to fatigue due to cyclic loads transferred through the implant whenever the patient is moving.

The second technique is to insert an implant with at least a partial osteoconductive surface into a bone cavity. Such an osteoconductive surface promotes ingrowth of the surrounding bone tissue. This eventually results in the implant being firmly attached to the bone tissue.

The process of bone ingrowth inter alia depends on the contact conditions between the bone tissue and the implant's surface. Contact conditions can be influenced by adapting the structural design of the implant, for example by altering its dimensions or surface structure to increase the contact surface to the cortical and/or cancellous bone tissue. Additional features such as ribs and grooves may also be used to fix the implant in the bone.

Typical materials used for such implants are titanium alloys, CoCr alloys and stainless steel. In this respect, titanium plays a vital role due to its biocompatibility and positive effect on osteoblasts in terms of creation of bone tissue on an implant's surface. As a result, titanium and in particular titanium alloys are frequently used as structural material for implants that substitute or are anchored in bone such as endoprosthetic implants.

Besides choosing the right material for these implants which has the required mechanical characteristics as well as biocompatibility, the implant's surface topography, i.e. the surface of the structural material of the implant body or any coating on its surface, also significantly influences the integration with bone tissue.

Improving osseointegration is often attempted by enhancing the contact stability through the design or by implant surface modification. Chemical and physical modifications of implant surface(s) aimed at increasing surface roughness are commonly used, resulting in higher micromechanical retention to improve primary and secondary implant stability. In addition to surface roughness, which is often relevant at the nano or micro level, the macroarchitecture of the implant is a factor for new bone growth. Bone growth stimulation has been demonstrated to be impacted by total porosity and pore sizes. However, the higher surface area inherent to porous structures also increases the risk for bacterial colonization, leading to implant infection, the second most common reason for failure of arthroplasty implants after aseptic loosening.

Hence, efforts have been made to equip implantable biomaterials with long-term antimicrobial properties. Due to the development of antibiotic resistance and the lack of long-term effects, drug-eluting strategies based on antibiotics are not optimal. Furthermore, surface topographical patterns on implants inhibiting bacterial adhesion have also gained interest, such as the use of nanotubules, nanopillars or nanocones.

Mechanisms to provide biomaterials with antimicrobial properties either rely on a bacteriostatic surface which passively inhibits bacterial adhesion and proliferation, or on bactericidal surfaces that deliver antimicrobial agents capable of killing bacteria. Metal ion coatings have been used as antimicrobial strategies for orthopedic implants.

Porous metallic implants offer a possible platform to improve osseointegration and minimize stress shielding. However, strategies to reduce the risk of bacterial adhesion and colonization of such implants are to be balanced against their potentially negative impact on osseointegration.

In sum, anchoring an implant without bone cement generally involves two phases that complement each other. In the first phase, a press-fit is applied for primary stability to initiate bone ingrowth and to create a strong interface between the bone tissue and the implant, which in turn provides secondary long-term stability. Such secondary long-term stability is the result of bone ingrowth, which is a solution sought for.

### SUMMARY

In accordance with one or more embodiments, devices and methods are provided.

The disclosure includes an implant configured to contact a portion of a bone, the implant including a stem having a proximal section and a distal section and a porous portion extending a distance along the stem in a longitudinal direction of the stem.

These and other advantages of the disclosure will be apparent to those of ordinary skill in the art by reference to the following detailed description and the accompanying drawings.

### BRIEF DESCRIPTION OF THE DRAWINGS

The accompanying drawings, which are incorporated in and constitute a part of this specification, illustrate embodiments of the disclosure, and, together with the summary given above, and the detailed description of the embodiments below, serve as a further explanation and disclosure to explain and/or illustrate embodiments of the disclosure.

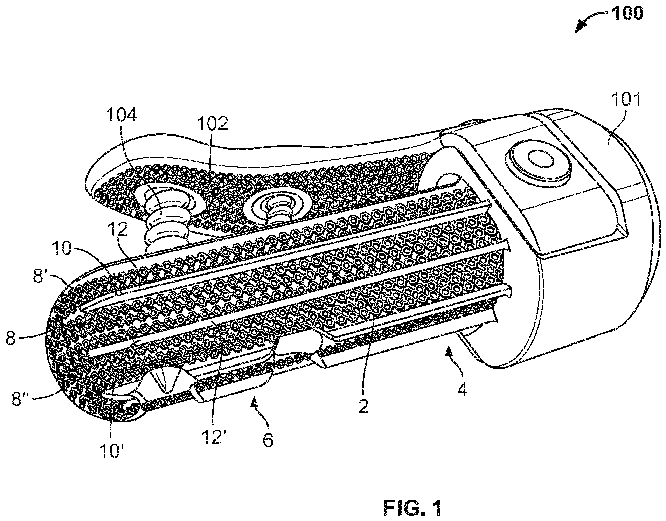

FIG. 1 is a perspective image of an implant of the present disclosure.

### DETAILED DESCRIPTION

To facilitate the understanding of this disclosure a number of terms of in quotation marks are defined below. It is noted that the drawings of the present application are provided for illustrative purposes only and, as such, the drawings are not drawn to scale. It is also noted that like and corresponding elements are referred to by like reference numerals.

In the following description, numerous specific details are set forth, such as particular structures, components, materials, dimensions, processing steps and techniques, in order to provide an understanding of the various embodiments of the present application. However, it will be appreciated by one of ordinary skill in the art that various embodiments of the present application may be practiced without these specific details. In other instances, well-known structures or processing steps have not been described in detail in order to avoid obscuring the present application.

It will be understood that when an element as a layer, region or substrate is referred to as being "on" or "over" another element, it can be directly on the other element or intervening elements may also be present. In contrast, when an element is referred to as being "directly on" or "directly over" another element, there are no intervening elements present. It will also be understood that when element is referred to as being "beneath" or "under" another element, it can be directly beneath or under the other element, or intervening elements ay be present. In contrast, when an element is referred to as being "directly beneath" or "directly under" another element, there are no intervening elements present.

As used herein, the term "substantially" or "substantial", is equally applicable when used in a negative connotation to refer to the complete or near complete lack of an action, characteristic, property, state, structure, item, or result. For example, a surface that is "substantially" flat would either be completely at, or so nearly flat that the effect would be the same as if it were completely flat.

As used herein, terms defined in the singular are intended to include those terms defined in the plural and vice versa.

As used in this specification and its appended claims, terms such as "a", "an" and "the" are not intended to refer to only a singular entity, but include the general class of which a specific example may be used for illustration, unless the context dictates otherwise. The terminology herein is used to describe specific embodiments of the invention, but their usage does not delimit the invention, except as outlined in the claims.

Unless otherwise indicated, all numbers expressing quantities of ingredients, properties such as molecular weights, reaction conditions, and so forth as used in the specification and claims are to be understood as being modified in all instances by the term "about." Accordingly, unless indicated to the contrary, the numerical parameters in the specification and claims are approximations that may vary depending upon the desired properties sought to be obtained by the present invention. At the very least, and without limiting the application of the doctrine of equivalents to the scope of the claims, each numerical parameter should at least be construed in light of the number of reported significant digits and by applying ordinary rounding techniques. Notwithstanding that the numerical ranges and parameters describing the broad scope of the invention are approximations, the numerical values in the specific examples are reported as precisely as possible. Any numerical value, however, inherently contains standard deviations that necessarily result from the errors found in the numerical value's testing measurements.

Thus, reference herein to any numerical range expressly includes each numerical value (including fractional numbers and whole numbers) encompassed by that range. To illustrate, reference herein to a range of "at least 50" or "at least about 50" includes whole numbers of 50, 51, 52, 53, 54, 55, 56, 57, 58, 59, 60, etc., and fractional numbers 50.1, 50.2 50.3, 50.4, 50.5, 50.6, 50.7, 50.8, 50.9, etc. In a further illustration, reference herein to a range of "less than 50" or "less than about 50" includes whole numbers 49, 48, 47, 46, 45, 44, 43, 42, 41, 40, etc., and fractional numbers 49.9, 49.8, 49.7, 49.6, 49.5, 49.4, 49.3, 49.2, 49.1, 49.0, etc. In yet another illustration, reference herein to a range of from "5 to 10" includes whole numbers of 5, 6, 7, 8, 9, and 10, and fractional numbers 5.1, 5.2, 5.3, 5,4, 5,5, 5.6, 5.7, 5.8, 5.9, etc.

In the discussion and claims herein, the tern "about" indicates that the value listed may be somewhat altered, as long as the alteration does not result in nonconformance of the process or structure to the illustrated embodiment. For example, for some elements the term "about" can refer to a variation of ±0.1%, for other elements, the term "about" can refer to a variation of ±1% or ±10%, or any point therein.

The present disclosure addresses objective problems noted herein and provides an implant that is able to create sufficient primary stability and at the same time has surface properties that promote bone ingrowth for long-term secondary stability.

In the present disclosure the implant refers to any element that can be implanted into a mammalian body and contact a bone of that mammal. For example, and without limitation, the implant of the present disclosure can be any portion of any hip implant/replacement, pelvic implant/replacement, shoulder implant/replacement, knee implant/replacement, ankle implant/replacement, elbow implant/replacement, wrist implant/replacement, finger implant/replacement, a revision prosthesis, jaw implants/replacements, dental implants/replacements, spinal implants/replacements, etc. In addition, the coating of the present disclosure can be on any portion of any surface of any device or element that can be implanted into a mammalian body and contact a bone of that mammal, including the examples listed above, as well as others.

The present disclosure provides an implant for implantation into and/or onto a portion of a surface of a bone. The implant comprises a stem having a proximal section and a distal section, at least one porous portion substantially extending along the longitudinal direction of the stem, and an optional ridge, the ridge being formed in between adjacent porous portions, when two or more porous portions are included.

The ridge can include an apex surface, with the surface roughness of the apex surface being lower than an external surface roughness of the porous portion, which can impact the objective of primary and secondary stability. The apex surface is the surface of the ridge facing away from the implant. More specifically, the lower surface roughness of the ridge's apex surface primarily causes elastic compression of the bone tissue during implantation and contact with a portion of the bone.

The implant can have at least two porous portions, a first porous portion and a second porous portion, with one ridge in between. The implant may also have three porous portions with two ridges in between, etc. with increasing number of porous portions and ridges as desired, with any of the porous portions and the ridges being present on any portion about the circumference of the stem.

The porous portion is configured to promote ingrowth of bone tissue and can have a higher average surface roughness in comparison to the apex surface of the ridge. The porous portion can be osteoconductive, e.g. it can be used by osteoblasts as a framework upon which to spread and generate new bone. After implantation and as part of the healing process, the bone tissue the implant is in contact with can start to attach itself and to grow into the topology provided by the porous portion. As a result, an increasing secondary stability supplements the anchorage achieved by the press-fit and provides a firm attachment of the implant to the bone.

The implant can be implanted to be in contact with any interior portion and/or exterior portion of any mammalian bone.

The porous portion can extend over any portion of the proximal section and/or the distal section of the stem.

An embodiment of an implant 100 of the present disclosure is shown in FIG. 1. This implant 100 is shown prior to an implantation to a mammal, with the implant 100 being configured to contact any portion of any bone of a mammal upon implantation.

The implant 100 includes a stem 2, with the stem 2 having a proximal section 4 and a distal section 6. In this embodiment the implant 100 also includes a supplemental stem 102, with the stem 2 and the supplemental stem 102 both being operably connected to a base 101. In this embodiment the relative angle between the stem 2 and the supplemental stem 102 can be modified by rotating inclined plane element 104 either clockwise or counter-clockwise to either expand or compress the relative angle between the stem 2 and the supplemental stem 102. In other embodiments the relative angle between the stem 2 and the supplemental stem 102 can be modified and/or fixed using any other suitable mechanical device and/or adhesive.

The supplemental stem 102 can include a plurality of pores, with the same or differing pore sizes. These pores and pore sizes are more fully discussed below.

The implant 100 includes a porous portion 8 that extends a distance along the stem 2, in a substantially longitudinal direction of the stem 2. In this embodiment three porous portions 8, 8' and 8" are visible, however in other embodiments, the implant 100 can include a single porous portion, or a plurality of porous portions around any section of the circumference of the stem 2, and along any section of either or both of the proximal section 4 and the distal section 6 of the stem 2.

The implant 100 also includes a ridge 10, which extends along the longitudinal direction of the stem 2, between a first porous portion 8 and a second porous portion 8' when the implant 100 includes at least two porous portions. The ridge 10 can include an apex surface 12, with the apex surface 12 being a surface of the ridge 10 that faces away from a central axis of the stem 2.

The ridge 10 includes a height dimension, such that the apex surface 12 is a distance away from a surface of the adjacent porous portion 8. This distance away can be any suitable distance, such as about 0.001 mm or less to about 10 mm or more, or about 0.01 mm to about 5 mm, or about 0.1 mm to about 3 mm, or about 0.5 mm to about 1.5 mm. This distance can be configured so that in embodiments where the implant 1 is configured to be implanted within an opening of a bone, such that the stem 2 extends a distance within an opening of the bone, the apex surface 12 can contact one or more portion of the internal bone surface while the porous portion 8 is present for purposes of bone ingrowth. In this embodiment, the apex surface 12 creates the primary stability for the implant 100 and the porous portion 8 is configured to provide secondary stability over time.

In some embodiments, an average surface roughness of the apex surface 12 can be lower than an average surface roughness of the porous portion 8. In some embodiments the average surface roughness of the apex surface 12 can be substantially the same as the average surface roughness of the porous portion 8 since in some embodiments both the ridge 10 and the porous portion 8 are formed of the same material.

The porous portion 8 and/or the ridge 10 can include a plurality of pores, with the same or differing pore sizes. Within the pores of the porous portion 8 and/or within pores of the ridge 10, there are free spaces interconnected with internal free spaces. The size of the pores and the interconnected, internal free spaces can be modified as desired.

Any portion of the implant 100 can be formed wholly or partially in any suitable way, such as through any suitable additive manufacturing (AM) and/or 3D-printing technology. For example, each of the supplemental stem 102, the porous portion 8 and/or the ridge 10 can be formed according to a suitable electron beam melting (EBM) process. EBM is an additive process for manufacturing and may produce solid or porous material. A powder of the desired material is provided in the desired granulometry. By the EBM process the powders of the desired material are deposited in successive layers at desired positions and in desired sequence (as defined in a preceding modelling step for the porous structure) and made to melt such as to form a coherent body, such as any portion of implant 100.

As one example of this AM technology, each of the porous portion 8 and/or the ridge 10 can be additively manufactured from Ti6Al4V extra low interstitial (ELI) powder in an electron beam machine (Arcam EBM Q10 plus).

The porous material of the supplemental stem 102, the porous portion 8 and/or the ridge 10 can be formed by "basic" elements, wherein a basic element is shaped like, for example, a tetrapod, but in other embodiments any suitable shape can be formed. A tetrapod is a structure having four legs being connected at a center point, each of the legs pointing away from the center point and spanning with their free ends, for example a tetrahedron, but in other embodiments different shapes can be formed. In one embodiment, the formed material of each of the porous portion 8 and/or the ridge 10 can be the porous structure TrabecuLink^{®}. In other embodiments, the porous material of the supplemental stem 102, the porous portion 8 and/or the ridge 10 can be formed into pores of any suitable shape, such as circular pores, substantially circular pores, rectangular pores, substantially rectangular pores, oval pores, substantially oval pores, any suitable polyhedron shape pore, an erratic shape pore, etc.

The tetrahedron, or any other suitable shape, may be irregular or regular. Optionally an isosceles tetrahedron is formed wherein each of the legs would form the same angle to each of the other three legs. In the context of the present disclosure a standard orientation of the tetrapod shall be one of the legs pointing upwards and the other three legs forming a stand, wherein the far ends of the three base legs define a base plane. This structure, or any other suitable structure can result in pore sizes of about 200 nm to about 5 mm or more, or about 250 nm to about 2 mm or more, or about 275 nm to about 1 mm or more, or about 300 nm or less to about 500µm or more, or about 350 nm or less to about 250 µm or more, or about 375 nm or less to about 100µm or more, or about 395 nm or less to about 10µm or more, or about 400 nm to about 1µm, or about 450 nm to about 800 nm, or about 500 nm to about 600 µm, and about 3 µm or less to about 20 µm or more (up to about 500 µm or more), or about 5 µm to about 10 µm surface roughness. The range of pore size and surface roughness can include any endpoint of any range noted above.

The desired material that forms any portion of the implant 100 can be modified for a desired outcome. In one embodiment, the material of the implant 100, including the supplemental stem 102, the porous portion 8 and/or the ridge 10 can be a titanium comprising material. The titanium comprising material can have at least some antimicrobial effects without substantially compromising osteocompatibility. In other embodiments, the titanium comprising material can further comprise aluminum and/or vanadium. One example of the titanium comprising material is Ti-6Al-4V.

In some embodiments an entire structure of the implant 100 can be formed through any suitable AM and/or 3D-printing technology. In other embodiments any suitable AM and/or 3D-printing technology can be used to apply one or more elements to an underlying skeletal structure. For example, an underlying skeletal structure can have the porous portion 8 applied thereto and/or the underlying skeletal structure can have the ridge 10 applied thereto, and/or the underlying skeletal structure can have the supplemental stem 102 applied thereto. Thus, a thickness of the porous portion 8, after being applied to an underlying skeletal structure, can be 1 mm or more, or 3 mm or more.

The described embodiments and examples of the present disclosure are intended to be illustrative rather than restrictive, and are not intended to represent every embodiment or example of the present disclosure. While the fundamental novel features of the disclosure as applied to various specific embodiments thereof have been shown, described and pointed out, it will also be understood that various omissions, substitutions and changes in the form and details of the devices illustrated and in their operation, may be made by those skilled in the art without departing from the spirit of the disclosure. For example, it is expressly intended that all combinations of those elements and/or method steps which perform substantially the same function in substantially the same way to achieve the same results are within the scope of the disclosure. Moreover, it should be recognized that structures and/or elements and/or method steps shown and/or described in connection with any disclosed form or embodiment of the disclosure may be incorporated in any other disclosed or described or suggested form or embodiment as a general matter of design choice. Further, various modifications and variations can be made without departing from the spirit or scope of the disclosure as set forth in the following claims both literally and in equivalents recognized in law.

## Claims

1. An implant (100) configured to contact a portion of a bone, comprising:
a stem (2) having a proximal section (4) and a distal section (6); and
at least one porous portion (8, 8', 8") extending a distance along the stem (2) in a longitudinal direction of the stem (2).

2. The implant (100) of claim 1, further comprising a ridge (10), wherein the porous portion (8, 8', 8") comprises at least two porous portions (8, 8', 8"), wherein the ridge (10) extends along the longitudinal direction of the stem (2) between a first porous portion (8) and a second porous portion (8') of the at least two porous portions (8, 8', 8").

3. The implant (100) of claim 2, wherein the ridge (10) comprises an apex surface (12), and the apex surface (12) has an average surface roughness that is lower than an average surface roughness of the porous portion (8, 8', 8").

4. The implant (100) of any one of claims 1 to 3, wherein a thickness of the porous portion (8, 8', 8") is 1 mm or more, preferably 3 mm or more.

5. The implant(100) of any one of claims 1 to 4, further comprising a supplemental stem (102) and a base (101), with the stem (2) and the supplemental stem (102) both being operatively connected to the base (101), wherein a relative angle between the stem (2) and the supplemental stem (102) can be modified and/or fixed by means of a mechanical device and/or adhesive.

6. The implant (100) of any one of claims 1 to 5, wherein the porous portion (8, 8', 8") comprises titanium.

7. The implant (100) of any one of claims 1 to 5, wherein the porous portion (8, 8', 8") comprises titanium, aluminum and vanadium.

8. The implant (100) of claim 7, wherein the porous portion (8, 8', 8") comprises Ti-6Al-4V.

9. The implant (100) of any one of claims 1 to 8, wherein at least a portion of the implant is formed by an additive manufacturing process.

10. The implant (100) of claim 9, wherein the porous portion (8, 8', 8") is formed by the additive manufacturing process.

11. The implant (100) of any one of claims 1 to 10, wherein the porous portion (8, 8', 8") comprises a plurality of pores with a pore size in the range of about 200 nm to about 5 mm.

12. The implant (100) of claim 11, wherein the pore size in in the range of about 275 nm to about 1 mm.

13. The implant (100) of claim 11, wherein the pore size in in the range of about 400 nm to about 1µm.

14. A method of manufacturing the implant (100) of any one of claims 1 to 13, comprising a step of forming at least a portion of the implant, preferably including the porous portion (8, 8', 8"), by an additive manufacturing process and/or 3D-printing technology.

15. The method of claim 14, comprising a step of forming one or more of, and preferably each of, the porous portion (8) and/or the ridge (10) by a suitable electron beam melting (EBM) process.
